# EUROPEAN PATENT APPLICATION

(11) **EP 2 600 268 A1**
(43) Date of publication of application: **05.06.2013**
(21) Application number: 13000569.7
(22) Date of filing: 17.11.2010
(51) Int. Cl.: G06F 19/00, A61M 5/142

(54) **Diabetes health management systems**

(30) Priority: 20.11.2009 US 622520
(62) Divisional of application: 10782199.3
(71) Applicant: Roche Diagnostics GmbH, 68305 Mannheim (DE); F. Hoffmann-La Roche AG, 4070 Basel (CH)
(72) Inventor: Galley, Paul J., Cumberland, IN 46229 (US); Price, John F., McCordsville, IN 46055 (US); Reinke, Robert E., Indianapolis, IN 46236 (US)

(57) **Abstract**

Diabetes health management systems comprising a portable devices having a user interface, a processor, a memory, and a communication circuit is disclosed. In one embodiment, a diabetes health management system has program code including a communications module, a data module, a therapy module, and an analysis module. The communications module wirelessly couples the portable device to a plurality of user devices. The data module receives and stores into the memory blood glucose measurement values, insulin dosage data, and health data entries. The therapy module determines a therapy advice message based at least in part on the received blood glucose measurement values, the received insulin dosage data, and displays the therapy advice message on the user interface. The analysis module displays on the user interface a graphical representation of selected blood glucose measurement values, selected insulin dosage data, selected health data entries, or combinations thereof.

## Description

### TECHNICAL FIELD

Embodiments of the present disclosure generally relate to diabetes health management systems and, more particularly, to diabetes health management systems residing in a portable electronic device.

### BACKGROUND

As background, persons with diabetes suffer from Type I or Type II diabetes in which the glucose level in the blood is not properly regulated by the body. As a consequence, many persons with diabetes often carry specialized medical devices for monitoring blood glucose (bG) levels and administering insulin. Such devices may include, for example, a blood glucose meter, a continuous blood glucose monitor, and/or an insulin pump.

Blood glucose meters commonly comprise a base unit that houses control and test electronics and a measurement strip receptacle that accepts a disposable measurement strip. One end of the strip is inserted into the measurement strip receptacle while an exposed area contains a reaction site in which the user deposits a drop of blood, which is often obtained by pricking the skin with a lancet. The test result is commonly displayed on the screen of the meter in milligrams per deciliter (mg/dL). Continuous blood glucose monitors commonly comprise a small patch that is worn under the skin and a receiver that displays the continuous blood glucose measurements. An insulin pump provides a user with programmable basal and bolus doses of insulin depending on the user's absorption of insulin, physical activity, food ingested and many other factors.

These specialized medical devices generate large amounts of data over the course of operation. Because proper insulin therapy depends on accurate information such as bG levels, insulin dosage rates and patterns, physical activities, a user's unique reaction to insulin and others, the data generated by these specialized medical devices and other electronic devices is of importance when providing an insulin therapy regimen to a person with diabetes. Currently this data is not collected in real time by a central device and is not used to make real time insulin therapy decisions. Real time collection of this data and appropriate action would be beneficial to providing accurate and effective insulin therapy.

### SUMMARY

It is against the above background that embodiments of the present disclosure provide diabetes health management systems that communicate with electronic devices, collect real-time data on a portable device carried by the user, provide insulin therapy to the user based on the real-time data, generate reports, and communicate data and reports to caregivers.

In one embodiment, a diabetes health management system comprises a portable device with a user interface, a processor, and a memory, The diabetes health management system includes program code which comprises a data module, a therapy module, and an analysis module. The data module causes the processor to receive and store into the memory blood glucose measurement values, insulin dosage data, and health data entries. The therapy module causes the processor to determine a therapy advice message based at least in part on the received blood glucose measurement values, the received insulin dosage data, and the health data entries stored in the memory, and display the therapy advice message on the user interface. The analysis module causes the processor to generate and display on the user interface a graphical representation of selected blood glucose measurement values, selected insulin dosage data, selected health data entries, or combinations thereof.

In a further embodiment the portable device comprises a communication circuit and the program code includes a communications module. The communications module causes the processor to control the communication circuit to wirelessly couple the portable device to a plurality of user devices, and display information provided by the user devices on the user interface.

In another embodiment, a diabetes health management system comprises a portable device having a global positioning module capable of providing a global positioning signal, a processor, a memory and a user interface includes program code further including a data module and a therapy advice module. The program code causes the processor to determine a present location of the user from the global positioning signal. The data module causes the processor to receive and store user health data in the memory, and generate and store a plurality of location profiles in the memory, each location profile having health data associated therewith. The therapy advice module causes the processor to display a therapy advice message on the user interface of the portable device, wherein the therapy advice message is based on a location profile corresponding with the present location of the user.

These and additional features provided by the embodiments of the present invention will be more fully understood in view of the following detailed description, in conjunction with the drawings.

### BRIEF DESCRIPTION OF THE DRAWINGS

The embodiments set forth in the drawings are illustrative and exemplary in nature and not intended to limit the inventions defined by the claims. The following detailed description of the illustrative embodiments can be understood when read in conjunction with the following drawings, where like structure is indicated with like reference numerals and in which:

FIG. 1 is a schematic representation of a portable device and diabetes health management system within a health care system according to one or more embodiments of the present disclosure;

FIG. 2 is a schematic diagram of a portable device according to one or more embodiments of the present disclosure;

FIG. 3 is a schematic diagram of a diabetes health management system according to one or more embodiments of the present disclosure;

FIG. 4 is a flowchart of a method of data collection and storage according to one or more embodiments of the present disclosure;

FIG. 5 is a schematic diagram of health data entries according to one or more embodiments of the present disclosure;

FIG. 6 is an illustration of a food database and food data entries according to one or more embodiments of the present disclosure;

FIG. 7 is a flowchart of a method of generating a food intake profile, generating an exercise profile, and providing a therapy advice message according to one or more embodiments of the present disclosure;

FIG. 8 is a schematic diagram of a plurality of location profiles according to one or more embodiments of the present disclosure;

FIG. 9 is a flowchart of a method of creating and populating a location profile with data according to one or more embodiments of the present disclosure;

FIG. 10 is a flowchart of a method of providing a structured reminder schedule according to one or more embodiments of the present disclosure;

FIG. 11A is an illustration of an event based report according to one or more embodiments of the present disclosure;

FIG. 11B is an illustration of an event based report according to one or more embodiments of the present disclosure; and

FIG. 12 is a flowchart of a method of shifting a therapy to a new time zone therapy schedule according to one or more embodiments of the present disclosure.

### DETAILED DESCRIPTION

Embodiments of the present disclosure relate generally to diabetes health management systems and methods of providing insulin therapy on a portable device. The diabetes health management systems and methods disclosed herein may be incorporated into a hand-held, portable device that a person with diabetes already uses, such as a cellular phone, personal data assistant, or music player, for example. Therefore, a user does not need to purchase an additional electronic device to carry.

Embodiments may provide real-time data collection from diabetes devices such as, but not limited to, blood glucose meters, continuous glucose monitors, and insulin pumps. Embodiments may also provide real-time and prospective insulin therapy guidance to Type 1 and Type 2 patients at the time of insulin dosing. The diabetes management systems described herein communicate with other devices in the system and act as a central hub of information management and user interaction by means of operational control and data display to and from the other devices. Some embodiments also communicate with caregivers of the patient's health support network, such as physicians, family members and emergency contacts, for example. Embodiments may provide the data and information needed so that the user may better manage his or her diabetes. The data collected by the system may be used to develop and refine a therapy provided to the user by use of pattern recognition, patient goals, detailed reports, and informed insulin dosage recommendations and reminders. Further, some embodiments may remotely control an insulin pump worn by the user in accordance with the collected data. These and other features of embodiments of the present disclosure will be discussed in detail below.

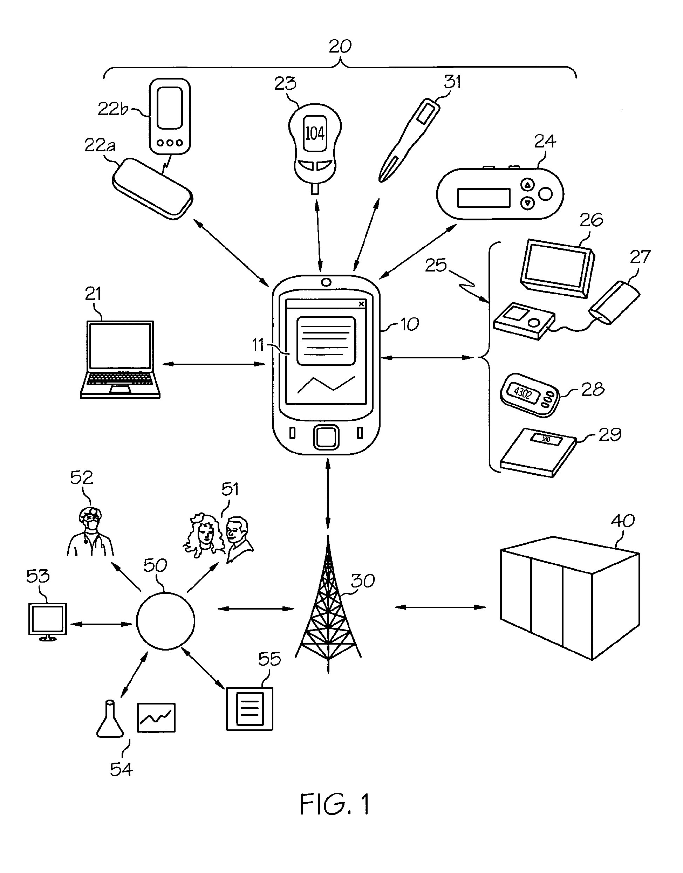

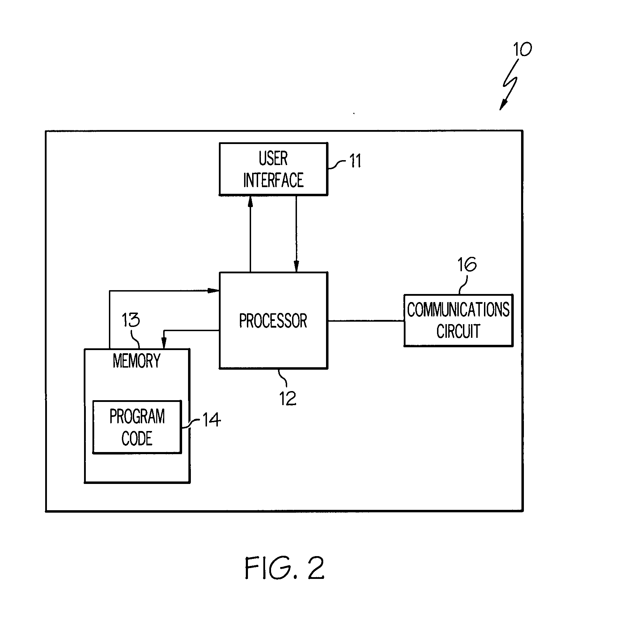

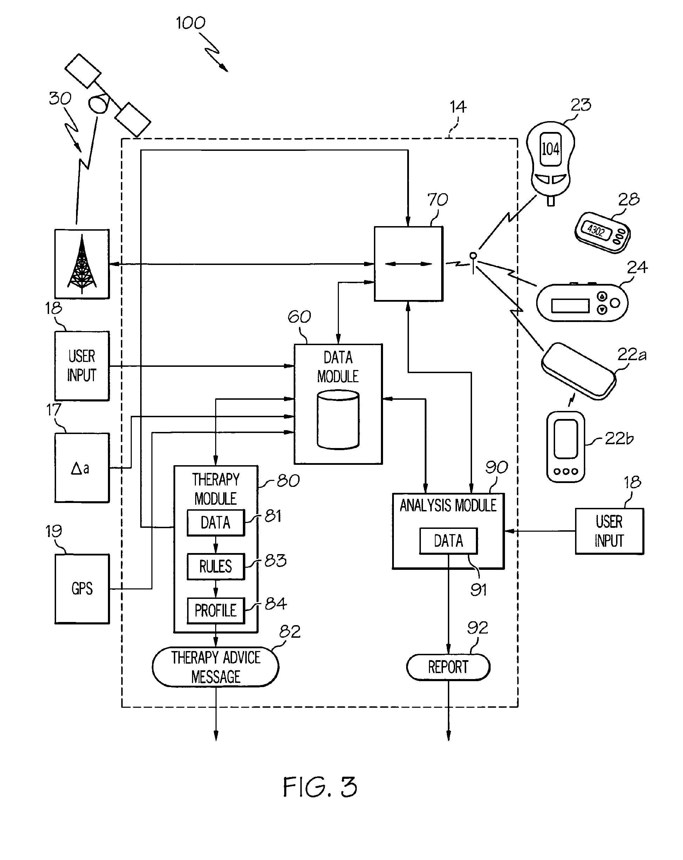

Referring initially to FIGS. 1 and 2, a portable device 10, such as a cellular phone, for example, is illustrated at the center of a healthcare system. A diabetes health management system 100 (see FIG. 3) is installed on the portable device 10 as program code 14 that resides in a memory 13 of the portable device 10. The memory 13 may be memory that is internal to the portable device 10 or a memory card that is inserted into the portable device 10. The portable device 10 comprise a user interface 11 that may be configured as a screen and a plurality of buttons and/or a keyboard. The portable device 10 further comprises a processor 12 that is operable to control the user interface 11 such that information may be displayed on the screen of the user interface. The processor 12 also is coupled to a communications circuit 16 that enables the portable device 10 to communicate with external devices. The communications circuit 16 may be configured to communicate using many wired and wireless communication protocols, such as Bluetooth, USB, infrared, IEEE 802.11, and/or proprietary wireless communication protocol, for example. As will be described below, the program code 14 comprises a plurality of modules that enable the processor 12 to effectuate the tasks of the diabetes health management system 100.

As illustrated in FIG. 1, the portable device 10, through the diabetes health management system 100, is coupled to a plurality of user devices comprising a plurality of diabetes devices 20, a plurality of electronic devices 25, one or more computers 21 and a communications network 30. The diabetes health management system 100 communicates with these user devices and the communications network 30 by either a wired or wireless connection, as described below. The plurality of diabetes devices 20 may include, but is not limited to, a blood glucose measuring device (e.g., a continuous blood glucose monitor patch 22*a* and receiver 22*b*, and a blood glucose meter 23) and an insulin administering device (e.g., an insulin pump 24 and an insulin smart pen 31). The plurality of electronic device 25 may include, but is not limited to, a global positioning system (GPS) device 26, a blood pressure measurement device 27 such as a sphygmomanometer, a pedometer 28, and a scale 29. These user devices may transmit generated data to the portable device 10 and the diabetes health management system 100 such that the diabetes health management system 100 is a central hub of health related data and information.

As shown in FIG. 1, the portable device 10 is coupled to a communications network 30. The communications network 30 may be a cellular network through which the portable device 10 may transmit and receive data. The communications network 30 may also be a wireless connection to the Internet, such as IEEE 802.11, or wired connection such as Ethernet. The communications network 30 may also be a satellite network in which data may be transmitted and received by the portable device 10.

Also coupled to the communications network 30 is a remote data storage location 40. The remote data storage location 40 may be a health network server that performs the function of a health data vault. Data and information collected from the plurality of diabetes devices 20, plurality of electronic devices 25, and computers 21 may be transmitted to the remote data storage location 40 via the communications network 30 and stored in a secured and encrypted manner. The data and information stored in the remote data storage location 40 may serve as a back up to the data and information that is stored in the memory 13 of the portable device 10. Further, the collected data and information may also be synched and stored on a user's personal computer 21. A user of the diabetes health management system 100 may configure access to the data and information stored in the remote data storage location 40 such that the user's caregivers may also have access to the data and information to further enhance diabetes therapy. Additionally, the user may have secured access to the data and information stored in the remote data storage location 40 through the communications network 30. The user may access the data and information through the portable device 10, a computer 21, or any other device that may be coupled to the communications network 30.

A healthcare network 50 is also coupled to the communications network 30. The health care network 50 enables caregivers such as physicians and healthcare professionals 52, family and friends 51, health care payers 55 (i.e., insurance company), a personal computer at the patient's home 53, and laboratory and research facilities 54 to access certain data and information collected by the diabetes health management system 100. The user may determine what data and information may be accessible to the various persons and entities that are a part of the healthcare network 50. For example, a user may decide to opt into a diabetes research program in which certain types of diabetes and health related data may be accessible to a research facility 54. Or a user may agree to provide a healthcare payer 55 access to particular progress reports generated by the diabetes health management system 100 such that the healthcare payer 55 may assess the value of the diabetes health management system 100. A healthcare professional 52 and family members 51 may also have access to the data and information. Further, as described in more detail below, the diabetes management system may be configured such that a healthcare professional 52 and/or family members 51 may receive messages regarding important diabetes or health related events, such as, but not limited to, hyperglycemic, hypoglycemic or ketosis states over the communications network 30. The messages may be in any form, such as by SMS text message, e-mail, telephone call, or dedicated electronic device.

FIG. 3 illustrates exemplary modules of the diabetes health management system 100 that are effectuated by the program code 14 residing in the memory 13 of the portable device 10. In the illustrated embodiment, the diabetes health management system 100 comprises a data module 60, a therapy module 80, an analysis module 90 and a communications module 70. The communications module 70 is program code 14 that interfaces with the communication circuit or circuits 16 of the portable device 10 such that the diabetes health management system 100 may communicate with the diabetes devices and electronic devices operated by the user. For example, a blood glucose meter 23 may transmit each blood glucose test result data to the portable device 10 via the communications circuit 16. The communications module 70 may then configure the test result data and provide it to the data module 60, which, as described below, may process the data and store it in a database residing in the memory 13. Similarly, the continuous blood glucose measurements provided by a continuous blood glucose monitor, e.g., patch 22*a* and receiver 22*b*, and insulin dosage data provided by an insulin pump 24 may be transmitted to the portable device 10 and stored in the memory 13. The communications module 70 also is configured to enable communication between the portable device 10 (and diabetes health management system 100) and the communications network 30.

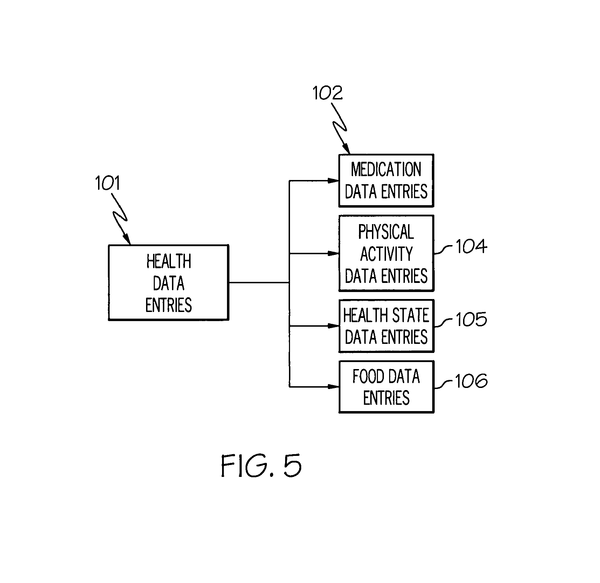

The data module 60 receives and processes the data and information from the various coupled devices. The data module 60 may mark or tag data that it receives with a temporal marker such as time and data. The received diabetes data may be blood glucose measurement values provided by a blood glucose meter 23 and/or a continuous blood glucose monitor 22*a*/*b* as well as insulin dosage data that is provided by an insulin pump 24, insulin smart pen 31, or user entry. The data may also comprise other information, such as health data entries 101. Health data entries 101 (see FIG. 5) may comprise health state and physical activity data such as stress level, exercise activity, illness, etc. as described in more detail below.

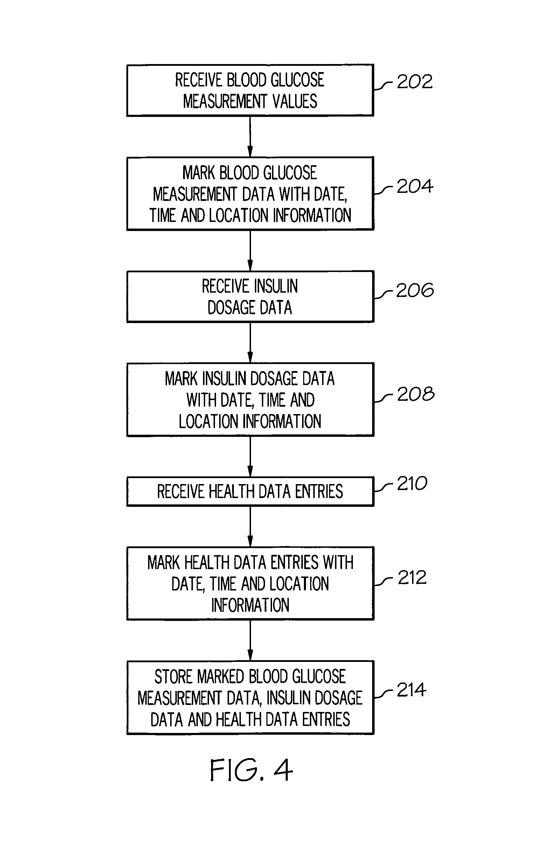

FIG. 4 is a flowchart that illustrates data collection and storage performed by the diabetes health management system 100. At block 202, the diabetes health management system 100 receives blood glucose measurement values at the data module 60. The blood glucose measurement values may be received automatically from a blood glucose meter 23 and/or a continuous glucose monitor 22*a*/22*b* via the communications module 70. For example, after the user performs a blood glucose test using a blood glucose meter 23, the test result may appear on the screen of the blood glucose meter 23 and also be wirelessly transmitted to the portable device 10 and diabetes health management system 100 through the communications circuit 16 and communications module 70 of the program code 14. The data module 60 may also receive the blood glucose measurement values retroactively by user input 18 through the user interface, for example (see FIG. 3). At block 204 the data module 60 receives the blood glucose measurement value or values and associates a date and time thereto. In embodiments in which a GPS signal is used to determine a location of the user, the detected location information may also be associated with the received blood glucose measurement value or values.

At block 206, the diabetes health management system 100 receives insulin dosage data by user input 18 through the user interface 11 and/or automatically from an insulin administering device (e.g., wirelessly), which may be an insulin smart pen 31, an insulin pump 24, or other similar devices. The insulin dosage data may comprise information regarding the details of the insulin administered to the user, such as bolus administration data (e.g., bolus shape, rate, timing, velocity, etc.) and basal rate data (e.g., basal rate pattern data, basal interval, basal rate time lag), for example. As described below, the diabetes health management system 100 may use this data to provide a graph of insulin delivery to the user. At block 208, the insulin dosage data may be marked with time and date information (and location information in some embodiments). The insulin data may be automatically marked with time and date information by the insulin administering device prior to being received by the diabetes health management system 100 or it may be marked by the diabetes health management system 100 upon receipt.

At block 210 the diabetes health management system 100 receives health data entries that are inputted into the portable device 10 by the user or determined automatically (e.g., physical activity detected by an accelerometer, hypoglycemia or hyperglycemia determined by blood glucose measurements). As described in detail below, health data entries reflect health-related data such as medication, physical activities, food intake, etc. A life mode selection may also be entered. A life mode is indicative of a life situation that the user is currently experiencing. Exemplary life modes that may be selected and entered by a user may include, but are not limited to, "meeting," "flight," "drive," and "night." The diabetes health management system 100 may use the user selected life mode to develop and provide a therapy message to the user, or alter insulin delivery. For example, a basal rate of insulin provided by the insulin pump 24 may be modified to a temporary basal rate when a user has selected "drive" to provide more insulin due to user inactivity during the drive. The health data entries and life mode selections may be marked with date, time and location data at block 212. At block 214, the marked blood glucose measurement values, the insulin dosage data, health data entries and life mode selections may be stored in the memory 13 of the portable device 10 by the data module 60. The blood glucose values, insulin dosage data and health data entries may be stored in memory right after receipt.

The data module 60 may also receive data from internal or external sensors and components as well as user-inputted data 18 that the user has inputted into the user interface 11. For example, the portable device 10 may comprise an accelerometer 17 that generates an acceleration signal that corresponds with a movement of the portable device 10. The data module 60 may be configured to receive and store the acceleration signal. The data module 60 may also be configured to process the received acceleration signal to determine a physical activity and an amount of energy consumed. For example, if the acceleration signal corresponds with a relatively low frequency of repeated movement, the data module 60 may determine that the user is walking. The data module 60 may then update the health data entries 101 to reflect the date, time, and duration of the walking session. Any type of physical activity may be detected from the acceleration signal, such as running, cycling, aerobics, etc. Additionally, the portable device 10 may also comprise a GPS circuit 19 that provides a location signal corresponding a location of the user. The data module 60 may receive the location signal and determine the location of the user. The data module may then associate a temporal marker to the location data and store it in the database residing in the memory. In an application where the portable device 10 does not comprises an internal accelerometer or GPS circuit, the communications module 70 may be configured to communicate with external accelerometer and GPS devices.

FIG. 5 illustrates a diagram of exemplary health data entries 101. Health data entries 101 correspond to health-related data (e.g., a health diary) that may be used by the therapy module 80 and analysis module 90 to provide therapy advice and/or generate reports. In the illustrated embodiment, the health data entries 101 is a database that is subdivided into four categories: medication data entries 102, physical activity data entries 104, health state data entries 105, and food data entries 109. It will be understood that more or fewer categories may be utilized. Health data entries 101 may be manually logged by the user using the user interface 11. As described above, health data entries 101 may also be generated automatically (e.g., detecting physical activity via an accelerometer 17).

Medication data entries 102 may include, but are not limited to, data entries associated with a type of medication taken by the user, the dosage amount, and the time of dosage. Medication data entries 102 may be entered by the user into the user interface 11. In some embodiments, a medication database may be stored in the memory of the portable device 10, or accessible on the Internet via the communications network 30. The medication database may comprise medical data relevant to particular types of medication. The user may use the medication database to select the type of medication taken, the dosage amount and the time of the dosage. The medication database may also comprise a picture of a typical medication label for a particular medication. The user may also be able to add custom medication database entries (as well as photos of a medication label) from which he or she may select medication data entries 102. In other embodiments, the user may manually enter the medication data entries 102. The diabetes health management system 100 may be programmed such that a user may enter a schedule of medication and dosage such that the medication data entries 102 are automatically added to the health data entries 101. The diabetes health management system 100 may further be programmed to prompt the user to verify whether he or she actually took the scheduled medication at the scheduled time. The user may select yes or no, or modify a medication data entry (e.g., change the time of dosage or dosage amount). After a medication data entry is created, the data module 60 may store it in an appropriate memory 13 location.

Physical activity data entries 104 are related to physical activities undertaken by the user. For example, physical activities may include, but are not limited to, walking, running, cycling, swimming, weight lifting, aerobics, etc. A user may enter a physical activity, the duration of the physical activity, and the time of the physical activity. Other information may also be provided in the physical activity data entry, such as calories burned (i.e., energy consumed), heart rate, etc. This information may be inputted by the user into the user interface 11, or obtained from an electronic device (e.g., a heart rate monitor) via the communications module 70. The user may select a physical activity and relevant information from a local or on-line database. The user may also add physical activities to the database.

As described above, physical activities may be automatically detected with the use of an accelerometer 17 and an associated acceleration signal. The acceleration signal may be used to detect a physical activity type, a physical activity intensity, a physical activity duration, or other similar physical activity characteristics. In one embodiment, the program code 14 may instruct the user interface 11 to display a message asking the user to verify the physical activity detected. The user may select yes or no, or modify the particulars of the detected physical activity. After the physical activity data entry is entered, either manually or automatically, the data module 60 may store it in an appropriate memory 13 location.

Data inputted into the health state data entries 105 relate to a health state or status of the user. Health states may include, but are not limited to, stress level, energy level, premenstrual, pregnancy, illness, etc. A user may enter a health state data entry when the user is in a particular health state. A health state database may be presented to the user on the user interface 11 in which the user may select the appropriate health state or states. The user may add user-defined health states to the health state database for future selection. A temporal marker may be manually or automatically applied to each health state data entry. After the health state data entry is entered, the data module 60 may store it in an appropriate memory 13 location. Health state data entries 105 may also be detected automatically. For example, instances of hypoglycemia and hyperglycemia may be automatically tagged as an event/health state data entry when the blood glucose measurement data is below/above particular thresholds.

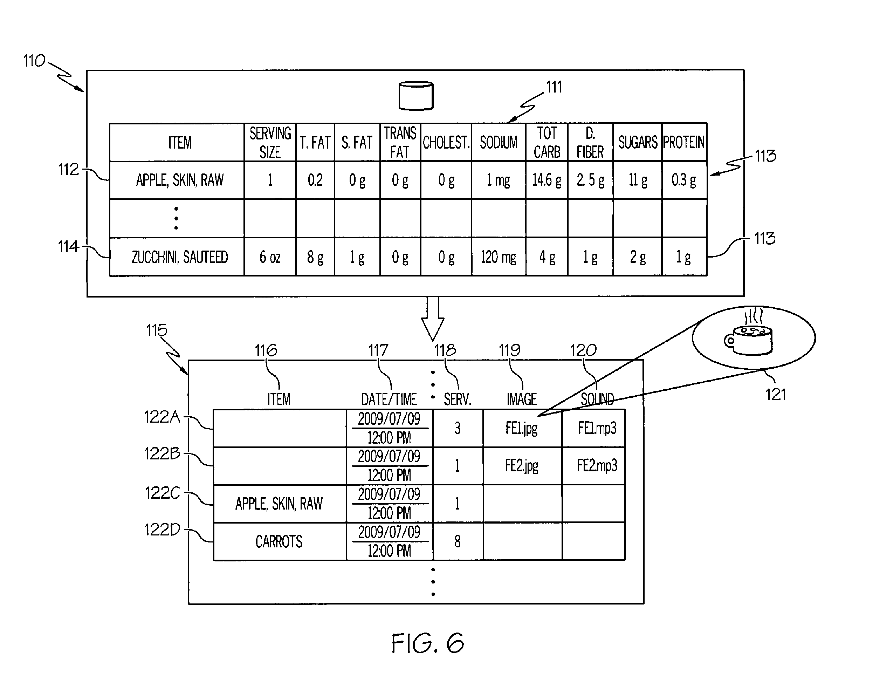

The food data entries 106 category of the health data entries 101 database relate to food items and related nutritional information of food consumed by the user. When a user consumes a particular food item, he or she may enter that food item and serving size information into the food data entries 106. The user may manually enter a food item and a serving size or may select a food item and serving size from a food database that resides in the memory of the portable device 10 or is located remotely and accessible via the communications module 70 and communications circuit 16. FIG. 6 is a schematic representation of a food database 110 and food data entries database 115 comprising food items selected by the user. The food database 110 comprises a plurality of food item categories 111 as illustrated by the heading of each column. The food item categories 111 may comprise the food item name, serving size, and nutritional attributes 113 such as total fat, saturated fat, total carbohydrates, etc. As an example, food item 112 is a raw apple with skin. The nutritional attributes 113 for one serving, which is one apple with skin, are provided. Sautéed zucchini is also provided as an example. The food database 110 may comprise many food items and associated nutritional attributes. The user may also add custom food items and attributes to the food database 110. The food database may contain food offerings from restaurant chains so that the user may have access to accurate food and nutritional data. In some embodiments, the user may mark a food item as a favorite food item. A list of the favorite food items may be displayed on the user interface 11 upon request so that the user may quickly select a favorite food item.

Before or after a user consumes a food item or items, he or she may access the food database 110 user through the user interface 11 of the portable device 10. The user may select a food item from the food database 110, enter a date and time of consumption and how many servings he or she consumed. In some embodiments, the user may take a photograph (e.g., using an internal camera feature of the portable device 10 or an external camera) of the food item and save it to the food entry. The photograph may be used by the user to enter the food item data at another time, or it may be transmitted to a caregiver such as a nutritionist for further analysis. Additionally, some embodiments may enable the user to create a voice recording that describes the food items that were consumed so that he or she may later enter the food item data at a more convenient time. FIG. 6 illustrates sample food items that were selected by a user. It will be understood that the food database 110 and food data entries database 115 are not limited to the configuration illustrated in FIG. 6 as more or fewer columns may be used, and the arrangement of the columns may be altered. Selected food item 122A, for example, does not have a name in the "Item" column 116. However, the user took a photograph 121 (FE1.jpg, shown in the "Image" column 119) with the portable device 10 and entered three servings (in the "Servings" column 118). The photograph 121 is associated with selected food item 122A in the "Image" column 119. The user also recorded voice recording as FE1.mp3 and associated it with food item 122A in the "SOUND" column 120. At a more convenient time, the user may access the photograph 121 and voice recording and be reminded that he or she should enter three cups of coffee for food item 122A. Similarly, image FE2.jpg and voice recording FE2.mp3 are associated with selected food item 122B. For selected food item 122C, the user used the database to select "Apple, Skin, Raw" as the food item. The user also entered 1 serving. Similarly, selected food item 122D comprises eight servings of "Carrots." Each selected food item within the food data entries is temporally marked with a date and time in the "DATE/TIME" column 117 provided by the user.

In one embodiment, a GPS signal provided by an internal GPS circuit 19 within the portable device 10 or an external GPS device 26 in communication with the portable device may be used to detect a location (e.g., a food serving establishment) of the user and provide food recommendations based on the present location of the user. As described in more detail below, the therapy module 80 may provide a therapy advice message to the user in the form of a food selection advice based on the food offerings of the detected food serving establishment, the diabetes data (e.g., blood glucose measurement values, insulin dosage data), and health data entries. Additionally, the food items offered by the detected food serving establishment may be initially and prominently displayed to the user on the user interface 11 when the user accesses the food database, or when the user enters the food serving establishment. In this manner, the specific food items offered by the food serving establishment are easy for the user to locate and select.

The selected food items may then be stored in a memory 13 location by the data module 60. For any of the data entry types discussed above, digital photographs and voice recording may be associated thereto. The data collected and stored within the memory 13 by the data module 60 may be accessed and viewed by the user via the user interface 11 of the portable device 10, a computer 21, or other similar devices.

Referring again to FIG. 3, the therapy module 80 is executable program code that retrieves and processes collected data 81 from the memory 13 of the portable device. The therapy module 80 may retrieve the data directly from the memory 13 or call on the data module 60 to retrieve and assemble the data 81. The therapy module 80 further comprises a plurality of clinical rules 83 that determine a particular therapy advice message based on the retrieved data 81, such as the stored diabetes (e.g., previous blood glucose measurement values) and health data (e.g., food data entries, physical activity entries). The therapy advice message 82 may comprise bolus recommendations, basal rate or premixed insulin recommendations, physical activity recommendations, etc. The clinical rules 83 may use the retrieved data 81 to determine an insulin dosage recommendation, for example. The user may also input insulin dosage parameters to further tailor the therapy advice recommendation (e.g., insulin sensitivity). The insulin dosage recommendation may comprise a particular basal rate that is based on the retrieved data 81 and applicable clinical rules or rules 83. When presenting the therapy advice message 82, the therapy module 80 may present relevant data to the user such that the user may understand the basis for the insulin dosage recommendation. For example, the therapy advice message may provide a bolus recommendation and also present current and past blood glucose values, total carbohydrates ingested, amount of active insulin, etc. The user may choose to accept or override the insulin dosage recommendation.

The diabetes health management system 100 may be programmed such that the user may retrieve and review past therapy advice messages on the user interface 11. Additionally, the user may retrieve and review relevant data associated with the recommendation as well as acceptance/override information. In this manner, the user may review past recommendations to determine if he or she agrees with a current recommendation and whether to accept or override/decline the recommendation given by the therapy advice message 82.

Further, as described below, the therapy module 80 may request one or more user profiles 84 from the data module 60 or retrieve the one or more user profiles 84 directly from the memory. A user profile 84 comprises historical data about the user and provides trending and predictive analysis. For example, a user profile may be directed toward a user's physical activity, insulin absorption, food intake at a particular location. The user profile 84 may consist of a single user profile that contains profile information such as food intake data, physical activity data, insulin response information such as the user's blood glucose response to insulin dosage as well as a location profiles, if desired. In other embodiments, the diabetes health management system 100 may develop separate profiles (e.g., a food profile, exercise profile, etc.) for the user.

The therapy module 80 may then use the user profile 84 to predict a user's behavior and response to insulin therapy. In this manner, the diabetes health management system may "learn" the user's behavior. Using the user profile 84 and the retrieved data 81, or only the user profile 84 where the profile contains all of the relevant data, the therapy module 80 may provide a therapy advice message 82 to the user. The therapy module 80 causes the program code 14 to instruct the user interface 11 to display the therapy advice message 82 automatically or upon user request. As illustrated in FIG. 3, the therapy advice message 82 may also be provided to the communications module 70 for transmission to the healthcare network 50 depending on user preferences. The therapy advice message 82 may contain information relating to a variety of health related topics, such as, but not limited to, insulin dosage recommendation, physical activity recommendation, food intake recommendation rest recommendation, insulin dosing reminders, and blood glucose test reminders.

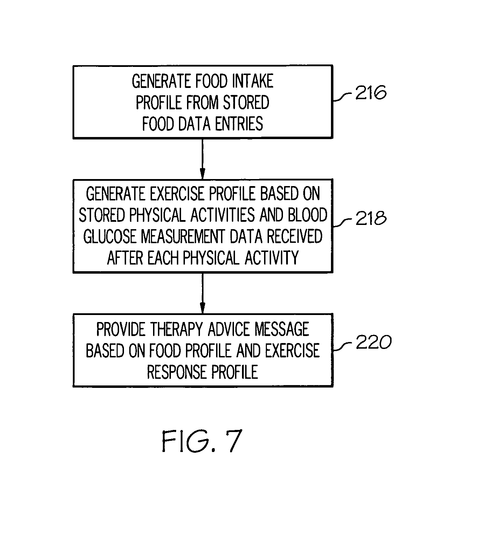

FIG. 7 depicts a flowchart that illustrates the development of exemplary exercise and food intake profiles for a user. At block 216, the data module 60 obtains food intake data from the memory 13 and stores or links the food intake data to a food intake profile. Over time, the diabetes health management system 100 may learn about the user's eating habits. The food intake profile is indicative of how a user may consume food products on a particular day. This food intake profile may then be used by the therapy module 80 to predict what a user may consume and determine what therapy advice message 82 to provide to the user, or how to adjust an insulin delivery provided by the insulin pump 24 (block 220). For example, the food intake profile may show that the user commonly eats foods high in carbohydrates and low in fat for lunch during the work week. The therapy module 80 may instruct the processor 12 to display a message on the user interface 11 that asks the user if he or she is or will be eating such foods for lunch. If yes, then the diabetes health management system 100 through the use of the communications module 70 may remotely control the insulin pump 24 to provide a bolus dose accordingly. Or, if the user does not use an insulin pump, the therapy module 80 may provide a therapy advice message 82 that recommends an insulin dosage accordingly.

At block 218, the data module 60 retrieves stored physical activity data entries and generates an exercise profile. The data module 60 may also retrieve blood glucose measurement data that is close to each physical activity data entry (e.g., blood glucose measurement data from initiation of the physical activity to two hours after completion of the physical activity) and adds this data to the exercise profile. In this manner, the diabetes health management system 100 may learn the exercise habits of the user (e.g., a three mile run every Monday, Wednesday and Friday mornings) as well as the blood glucose response due to the physical activity. At block 220, the therapy module 80 may use the exercise profile to develop and provide a therapy advice message 82 such issuing a blood glucose reminder, or remotely control the insulin pump 24 to provide an insulin dose in accordance with a predicted physical activity or a recently undertaken physical activity. For example, if a user normally goes on a Monday morning run, the therapy module 80 may issue a therapy advice message 82 with bolus advice, basal or pre-mix insulin advice in accordance with the user exercise profile. Or, the user may input a physical data entry corresponding to a weight lifting session. The therapy module 80 may use the exercise profile to look up the user's response to weight lifting and provide a therapy advice message 82 to the user accordingly. The blood glucose measurement data after each physical activity may be linked to or stored in the exercise profile so that the diabetes health management system 100 may continuously learn the blood glucose responses to the various physical activities.

The diabetes health management system 100 may also be programmed to generate location profiles that contain diabetes and health related data associated with a location of a user. A user's food intake, physical activity level, reaction to insulin, stress level, etc. may vary from location to location. For example, the physical activity level of a user at his or her workplace may be lower than at his or her home if the user works at a desk for the majority of his or her workday. Therefore, the basal rate or rates necessary at work during the work day may be different than the basal rate or rates necessary at home on the weekend.

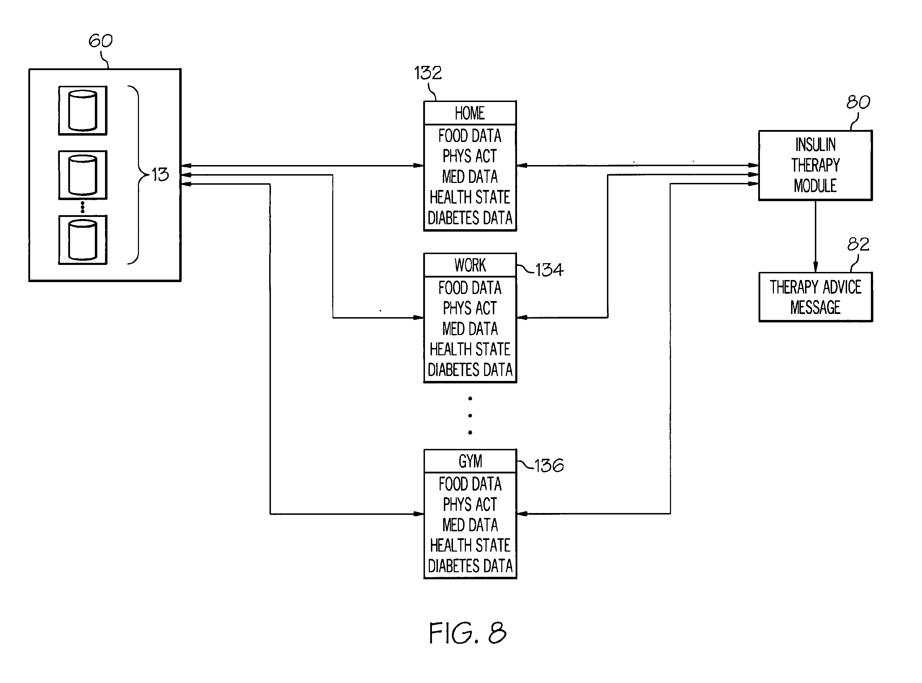

Referring now to FIG. 8, as part of or supplemental to the user profile 84, a home location profile 132, a work location profile 134, and a gym location 136 are illustrated. It will be understood that more or fewer location profiles may be used. The user may develop custom location profiles for locations that he or she frequently visits. The data module 60 retrieves data from the memory 13 of the portable device 10 and populates the illustrated location profiles 132, 134, 136 accordingly. The data linked to the location profiles 132, 134, 136 may comprises food data, physical activity data, health state data, and diabetes related data associated with the particular location.

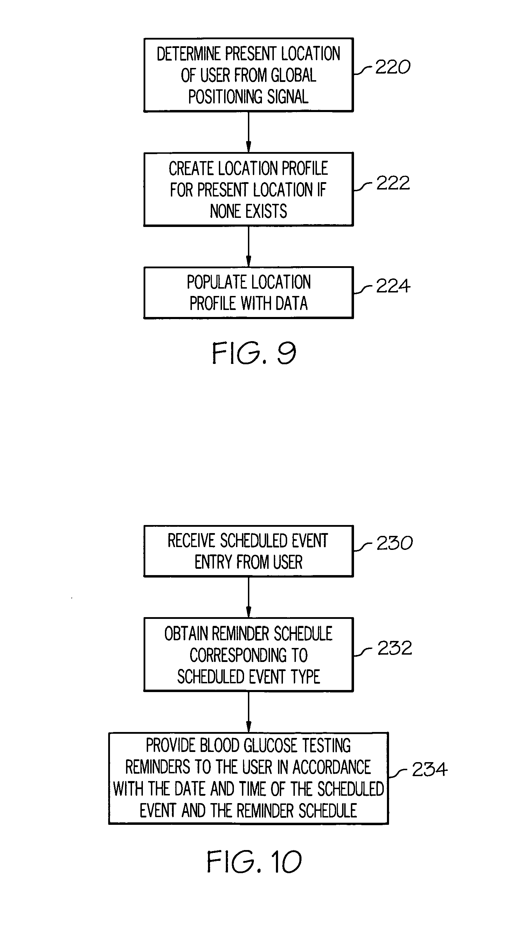

The location of the user may be provided by the user via the user input 11 or determined automatically by a GPS signal provided by an internal GPS circuit 19 or an external GPS device 26. FIG. 9 depicts of flowchart of detecting a present location of the user and developing a user profile for that location. At block 220, the diabetes health management system 100 is programmed to determine the present location of the user by receiving a GPS signal. If no location profile exists for the present location, a new location profile for the present location may be created at block 222. The user may be given the option to create a new user profile or instruct the diabetes health management system 100 to create a new location profile in accordance with user-defined parameters (e.g. only create a new profile for a frequently visited location or for a location at which the user stays for a certain duration). The user may also manually create a new location at any time.

At block 224, data (e.g., blood glucose measurement values and health data entries) associated with the location may be stored in the appropriate location profile. For example, all of the food that a user eats, the physical activity undertaken, blood glucose measurement, etc. at a particular location may be stored in the respective user profile. The therapy module 80 may use these location profiles to predict user behavior, health and blood glucose responses at each location and provide a therapy advice message 82 accordingly. In this manner, the diabetes health management system 100 may know the user's lifestyle patterns at various locations. A user may be more active on the weekend while at home by doing activities such as yard work and house work and may therefore require less insulin than when the user is at work. Conversely, a user that works in a field that requires manual labor may need less insulin while at work than when he or she is at home. The therapy module 80 may make these insulin dosing recommendations by using the location profiles.

The therapy module 80 may be programmed to provide a therapy advice message 82 in the form of one or more reminders. The reminders may be presented to the user by way of text or graphical displays (e.g., icons) provided on the user interface 11 or customizable audio reminders such as tones, songs or user-recorded voice recordings. The reminders may include, without limitation, blood glucose testing reminders (e.g., post prandial, episodic testing), insulin dosing reminders, medication reminders, and physical activity reminders. The reminders provided by the therapy module 80 may match the reminders/warnings issued by the plurality of diabetes devices 20. For example, the insulin pump 24, which may be in communication with the diabetes health management system 100, may provide a reminder to the user. The therapy module 80 may be programmed to provide an insulin pump 24 reminder on the user interface 11 that uses the same reminder terminology as issued by the insulin pump 24.

Reminders may be configured as time based or event based. Exemplary event-based reminders may include, without limitation, blood glucose test reminders after a hypoglycemic event, a hyperglycemic event, or after a meal. For example, the user may configure and receive a hypo/hyper blood glucose re-test reminder including relevant data triggered by a high or low blood glucose result either from a blood glucose meter 23 or a continuous glucose monitor 22*a*/*b*. The therapy module 80 may also be configured to accept user-programmed reminders. The user may select and/or define the time-based and event-based reminders. Reminders may be a single reminder or repeatable. Non critical reminders may be dismissed or postponed by the user. Reminders may be suspended if the reminder action was performed within a period of time prior to the reminder time (e.g., a user tests his or her blood glucose fifteen minutes prior to the reminder time).

The therapy module 80 may also be programmed to provide structured reminders to the user based on a scheduled event entry. The structured reminders may alter a normal reminder and insulin therapy schedule. For example, if a user has an appointment with his or her doctor, the doctor may wish to alter a testing schedule to have more blood glucose measurements during certain parts of the day. Or, as another example, if a user is determining his or her morning basal requirements, the therapy module 80 may issue a reminder to the user on the user interface that reminds the user to not eat breakfast and to test his or her blood glucose regularly until lunchtime. FIG. 10 is a flow chart illustrating the structured reminder process. At block 230, the user enters a scheduled event into the user interface 11 where it is then stored in the memory 13. The therapy module 80 may then obtain a reminder schedule based on the type of scheduled event at block 232. The therapy module 80 then issues reminders to the user in accordance with the reminder schedule and the date and time of the scheduled event at block 234.

In some embodiments, the therapy module 80 may be programmed to issue warnings to the user on the user interface 11 or by audio signals. The warnings may be based on a warning being provided by a diabetes device such as an insulin pump 24, an insulin smart pen 31, a blood glucose meter 23, or a continuous glucose monitor 22*a*/22*b*, for example. Such warnings may relate to device maintenance, device errors, device malfunctions, low battery power, etc. Warnings may also be issued when the user selects a basal rate profile that is empty and does not contain data. The user may prompted to edit the basal rate profile. Therefore, the user may obtain information and warnings relating to the plurality of diabetes devices 20 (and other devices) from the portable device 10.

Additional warnings may include, for example, hypoglycemic or hyperglycemic warnings if blood glucose measurements are below/above customized thresholds. These warnings may be configured as an emergency alarm. The warning may comprise a visual graphic on the user interface 11 and/or an audible tone. A warning may also be provided after a missed blood glucose test after a bolus was requested without a blood glucose result received by the portable device 10. It will be understood that other warnings may also be issued.

To further aid the user in managing his or her diabetes, the therapy module 80 of the diabetes health management system 100 may also be programmed to allow the user to set goals relating to various health parameters so that the user may attempt to meet such user-defined goals. For example, goals may include energy burned, number of steps taken, blood pressure values, calories, carbohydrates, fat, or protein ingested, glycemic variability, cholesterol/lipids, etc. The therapy module 80 may be programmed to present health parameter values in tables, graphs, messages, etc. so that the user may track his or her progress in accomplishing the goal or goals. The goal setting feature may also be incorporated into a game that includes educational information to aid the user in managing his or her diabetes and health in general. The user may also opt-in to receive periodic advice/feedback messages regarding the user's progress versus his or her entered goals.

Referring now to FIGS. 2 and 3, the communications module 70 of the diabetes health management system 100 and communications circuit 16 of the portable device 10 may cooperate to remotely control and configure the plurality of diabetes devices 20 and the plurality of electronic devices 25 by sending command signals to such devices. The user may use the diabetes health management system 100 to set up, control, program, etc. the plurality of devices from the portable device 10. The system 100 may request, receive and store relevant data from the plurality of diabetes devices 20 and the plurality of electronic devices 25.

For example, the diabetes health management system 100 may be utilized to remotely configure and control an insulin administering device, such as an insulin pump 24 or insulin smart pen 31. The user may set up the insulin administering device with an insulin dosage. The user may be able to select the dosage amount from a list of insulin dosage step size units (e.g., whole, half or quarter units). The user-selected dosage amount may be wirelessly sent to the insulin administering device.

The user may be able to use the diabetes health management system 100 and portable device 10 to control pump functions of the insulin pump 24. The system 100 may be programmed to wirelessly send user-defined bolus delivery commands to the insulin pump 24. The bolus delivery commands may be generated automatically upon the user's acceptance/override of the therapy advice message 82 provided by the therapy module 80. The user may also use the system 100 to adjust bolus delivery properties of the insulin pump 24 such as bolus delivery velocity from the portable device 10. The user may also adjust a time lag for the initiation of a bolus. The diabetes health management system 100 may also control the insulin administering device in accordance with user-defined basal rate profiles. The user may define time intervals for basal rate profiles (e.g., definable time blocks in 15 minute intervals), and define an adaptive bolus to be administered by the insulin pump 24. An adaptive bolus may be defined as a combination of several extended boli administered within a set time frame. The adaptive bolus may modulate a pre-defined postprandial insulin infusion based on food recently consumed by the user.

In addition to remotely controlling the insulin pump 24, the diabetes health management system 100 may be programmed to receive data from the insulin pump 24 (or other insulin administering device such as an insulin smart pen 31) such that the data may be viewed on the user interface 11 of the portable device 10. The system 100 may configure and display this data such that the user may graphically view the progress of the current bolus delivery amount, review active insulin data, and historical insulin pump data graphs and/or table formats. Insulin administration data received from the insulin pump 24 may be presented to the user on the user interface 11 in a user-selectable time range (e.g., one minute, five minutes, ten minutes, etc.)

The diabetes health management system 100 may also be utilized to control and configure other diabetes devices 20 (and electronic devices 25) in which the portable device 10 may be electronically coupled.

The diabetes health management system 100 may be programmed to notify caregivers of warnings, blood glucose measurements, reminders, and other data via the communications module 70. The method of notification may include, but not limited to, SMS text message, e-mail, and automated telephone call. The method of notification may depend on the particular caregiver and the severity/urgency of the notification. The user may define which caregivers (e.g., mother, father, physician, etc.) receive what type of notification (e.g., hyperglycemic condition, ketosis, missed blood glucose measurement, etc.). The information contained within the notification may be defined by the user.

Referring once again to FIG. 3, an analysis module 90 is configured to generate reports 92 and analysis based on the collected data such as the blood glucose measurement data, insulin dosage data and health data entries. The analysis module 90 may call on the data module 60 to retrieve the requested data 91 for the generation of the reports 92, or it may retrieve the desired data 91 directly from the memory 13 of the portable device 10. The reports 92 may be tailored to any health condition, and may link various health conditions together. The reports may comprise information relating to diabetic condition, insulin dosage, bG measurements, heart rate, physical activity, body weight/mass, food intake, and many others. A report 92 may be generated based on user input 18, such as when a user enters a report request using the user interface 11. Reports 92 may also be generated on a schedule as defined by the user or a caregiver. In some embodiments, a caregiver may request a report from the diabetes health management system 100 through the use of the communications network 30. Depending on the preferences of the user, the analysis module 90 may generate the requested report and the communications module 70 may transmit the requested report to the caregiver via the communications network. The generation and transmission of reports may be automatic in accordance with a schedule, or the caregiver may request reports from the user. The report may be presented to the user on the user interface 11 of the portable device 10 or on a computer 21 screen. The caregiver and user may concurrently review, analyze and discuss the report to further tailor the diabetes management.

The reports generated by the analysis module 90 may comprise graphical representation of health related data, and may be presented in table and/or graph format. The health related data may comprise data from a blood glucose meter 23, continuous glucose monitor 22*a*/22*b*, insulin pump 24, food data entries 106, physical activity entries 104, etc. Reports may display average health related data values over a selectable period of time, as well as indicate trends of health related data. Other reports may display a snapshot of blood glucose measurement data over a particular range of time (e.g., a three-day snapshot).

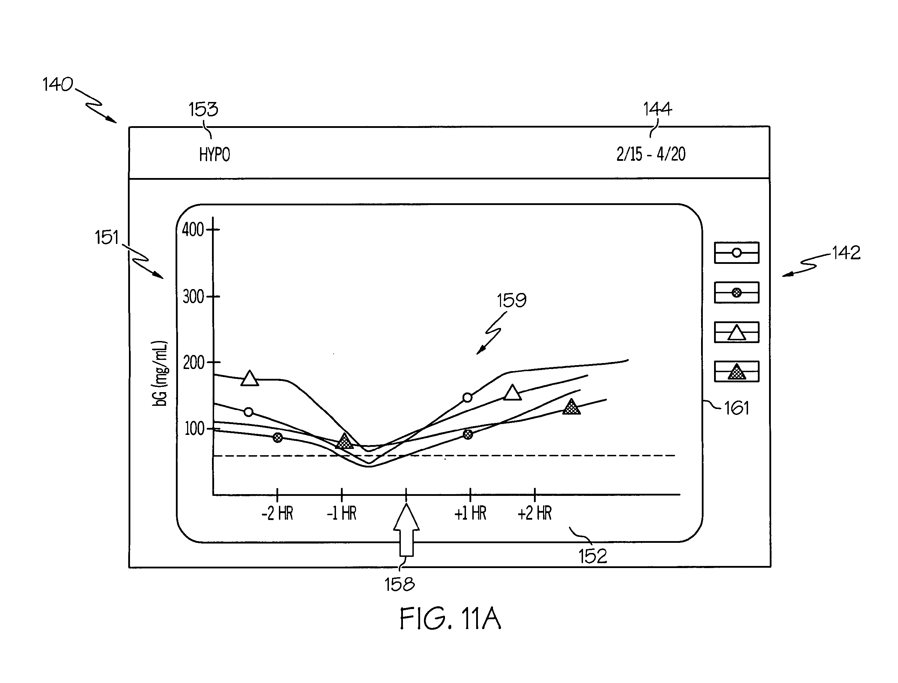

Reports may also be event based. Events may be contained in the health data entries (e.g., physical activity data entries, medication data entries, health state data entries, and food data entries). For example, a user may select a particular event to generate a report depicting relevant data near in time to one or more multiple occurrences of that event in a table or graphical format. An exemplary report 140 displayed on a user interface 11 of a portable device 10 is illustrated in FIG. 11A. It will be understood that reports generated by the analysis module 90 are not to be limited to the content and configuration of the exemplary reports included herein. The report 140 is based on a hypoglycemic event that was logged by the diabetes health management system 100. The hypoglycemic event is indicated by an event tag 153, and a date range 144 indicates the range of time displayed in the graph. The report 140 comprises a graph area 161, an independent axis 152 (the x-axis) and a dependent axis 151 (the y-axis). The independent axis 152 is scaled in accordance with the time that occurred before and after an event. Arrow 158 represents when the event occurred (i.e., pre-event period and post-event period). The dependent axis 151 comprises a label indicating the data type being illustrated, and may be blood glucose values, carbohydrates, protein, energy burned, etc. Within the graph area are data sets 159 that represent the data over time, and the labels 142 indicate the individual data sets.

According to the exemplary report 140, four instances of hypoglycemia were tagged as events within the date range of February 15th through April 20th. The four hypoglycemic events are represented by the data sets 159 within the graph area 161. The hypoglycemic events were tagged when the blood glucose of the user fell below 70 mg/dL. A user may use this report 140 to evaluate how quickly he or she entered a hypoglycemic state and how quickly he or she recovers. Reports relating to other events may also be generated, such as hyperglycemia, exercise, illness, stress, menstrual state, food intake, ketosis, etc. As described above, the events may be tagged manually by the user or detected automatically.

Reports may also combine and present to the user multiple, related data sets. These reports may provide cause and effect analysis to the user so that the user may determine what actions he or she took to lead to a particular event or condition. For example, a user may generate a hypoglycemic report that also provides health data near in time to a particular hypoglycemic state that the user experienced. This information may aid the user in learning about how he or she enters a hypoglycemic state as well as effective actions to get out of a hypoglycemic state.

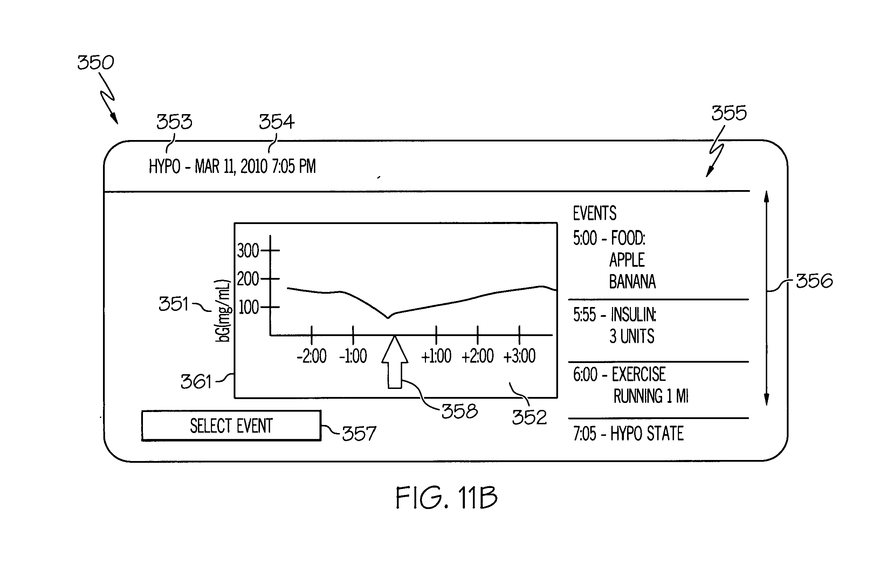

FIG. 11B illustrates an exemplary hypoglycemia report 350 that includes data temporally related to a hypoglycemic event. The report 350 comprises a graph area 361 having an independent axis 352 (time) and a dependent axis 351 (blood glucose measurements in mg/dL). The report 350 is directed to a hypoglycemic event occurring at 7:05 PM on March 11, 2010 as indicated by event tag 353 and date tag 354. Arrow 358 also indicates the time in which the hypoglycemic event occurred. The illustrated report 350 plots blood glucose values over a range of time that is between three hours before the hypoglycemic event and three hours after the hypoglycemic event. The size of the range displayed by the report 350 may be adjusted. The report 350 further comprises an event log 355 that lists the health data entries/events that occurred within the selected time range. In the illustrated embodiment, the event log 355 provides the time and type of health data entry/event (e.g., the user ate an apple and a banana at 5:00 PM). Additional event based reports may be generated by pressing the Select Event button 357. The user may use the scroll device 356 to scroll forward and backward in time. The user may use this type of report to perform a cause and effect analysis that promotes learning from past events and helps improve present and future results.

The reports generated by the analysis module 90 may be printed to a printer, exported to an external program (e.g., Microsoft Excel) for further analysis, saved for later access and/or transmitted to caregivers. The analysis module 90 may be programmed to compile caregiver-specific reports. For example, reports may be tailored toward family, health care professions such as physicians, and health care payers. The reports transmitted to family and health care professionals may aid in supporting the user with his or her diabetes, while reports transmitted to health care payers may enable the health care payer to view the progress and success of the user under the insulin therapy provided by the diabetes health management system 100. Reports may be sent to the user's caregiver so that the user and the caregiver may remotely and concurrently review the report together to discuss the user's progress and condition.

The diabetes health management system 100 may also be programmed to shift an insulin therapy from a first time zone schedule to a second time zone schedule. As diabetes management may be schedule-dependent, it may be desirable to shift the therapy such that the advice presented to the user is incrementally shifted from the first time zone to the second time zone. For example, if a user travels from New York to Los Angeles, the therapy module 80 may be programmed to incrementally shift from the Eastern Time Zone to the Pacific Time Zone by gradually moving the reminders of when to eat, when to dose insulin, how to adjust basal rates, etc. such that after a period of time (e.g., three days) the user is accustomed to the Pacific Time Zone. The user may define a time zone change profile that instructs the diabetes health management system 100 how to shift the insulin therapy. For example, the user may input how many days he or she will be in the new time zone, how quickly he or she desires to shift to the new time zone, what type of schedule he or she intends to adhere to (e.g., meetings, physical activity), etc. such that the therapy module 80 may provide therapy advice messages accordingly.

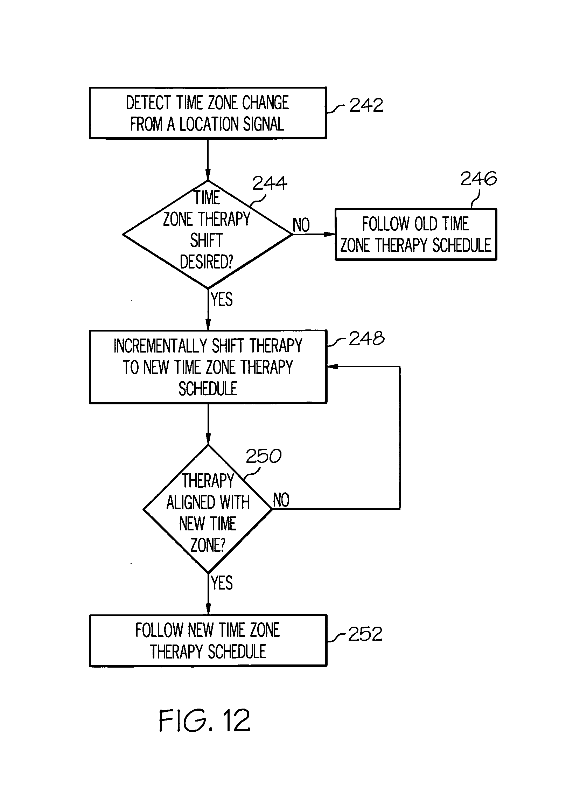

FIG. 12 is a flowchart depicting how the therapy module 80 may shift the user's insulin therapy from a first time zone therapy schedule (i.e., an old time zone therapy schedule) to a second time zone therapy schedule (i.e., a new time zone therapy schedule) according to one embodiment. At block 242, the diabetes health management system 100 automatically determines a location of the user from a location signal provided by an internal GPS circuit 19 or a GPS device 26. The diabetes health management system 100 may determine if a time zone change has occurred by comparing a present location with a recent location. The user may also enter a new location into the diabetes health management system 100 manually rather than the use of automatic detection.

After the location of the user and the new time zone is determined, the user may be prompted to decide whether or not to shift the insulin therapy to the new time zone therapy schedule at block 244. If the user is only going on a short trip, he or she may not elect to shift the therapy schedule, for example, and may then follow the old time zone therapy schedule at block 246. If the user elects to shift the therapy schedule, the insulin therapy may be incrementally shifted to the new time zone therapy schedule over time, which may be over the course of several days (block 248). The insulin therapy is incrementally shifted (blocks 250 and 248) until the insulin therapy is aligned with the new time zone. The therapy module 80 then provides therapy advice messages in accordance with the new time zone therapy schedule at block 252.

The diabetes health management system 100 may be programmed to provide additional therapy related features to the user. For example, the diabetes management system 100 may connect to the Internet through the communications module 70 and communications circuit 16 to provide educational diabetes and health training videos in an embedded web browser. The training videos may provide therapy guidance on a wide variety of topics and levels of skill, such as how to inject insulin, how to treat a hypoglycemic episode, or new products and technologies that may be available. The embedded web browser may also provide the user access to various diabetes related message boards and social networking sites that allow patients with diabetes to collaborate with one another. The diabetes health management system 100 may also wirelessly connect to the Internet to provide access to information regarding nutritional content of food, information regarding pharmaceuticals, etc.

It should now be understood that diabetes health management systems described herein may be operable to provide real-time diabetes and health related data collection and storage. Embodiments may couple user devices to a central, portable device for data collection and communication. The data may be wirelessly (or by wired connection) obtained from medical devices such as blood glucose meters, continuous glucose monitors, and insulin pumps, and other electronic devices such as pedometers, pulse sensors, and blood pressure monitors. Embodiments may also provide real-time and prospective insulin therapy guidance to users based on the collected data and hardware provided within the portable device. Data may be shared with caregivers to monitor and provide support to the user. Embodiments may generate and transmit custom reports based on the collected data, as well as remotely control electronic devices.

It is noted that recitations herein of a component of a particular embodiment being "programmed" in a particular way, "configured," "programmed" or "operable" to embody a particular property, or function in a particular manner, are structural recitations as opposed to recitations of intended use. More specifically, the references herein to the manner in which a component is "programmed," "configured" or "operable" denotes an existing physical condition of the component and, as such, is to be taken as a definite recitation of the structural characteristics of the component.

Having described the invention in detail and by reference to specific embodiments thereof, it will be apparent that modifications and variations are possible without departing from the scope of the invention defined in the appended claims. More specifically, although some aspects of the present invention may be identified herein as preferred or particularly advantageous, it is contemplated that the present invention is not necessarily limited to these preferred aspects of the invention.

The following is a list of possible embodiments of the invention: Embodiments of the present invention may be provided with features described herein individually, or in any suitable combination, whether or not specifically described in combination based on the teachings herein.
1. A diabetes health management system for use in a portable device having a user interface, a processor, a memory, and a communication circuit, the diabetes health management system comprising program code further comprising a communications module, a data module, a therapy module, and an analysis module, wherein:
   the communications module causes the processor to control the communication circuit to wirelessly couple the portable device to a plurality of user devices, and display information provided by the plurality of user devices on the user interface;
   the data module causes the processor to receive and store blood glucose measurement values, insulin dosage data, and health data entries into the memory;
   the therapy module causes the processor to determine a therapy advice message based at least in part on the blood glucose measurement values, the insulin dosage data, and the health data entries stored in the memory, and display the therapy advice message on the user interface; and
   the analysis module causes the processor to generate and display to a user on the user interface a graphical representation of selected blood glucose measurement values, selected insulin dosage data, selected health data entries, or combinations thereof.
2. The diabetes health management system of embodiment 1 wherein the communications module further causes the processor to transmit to a health network server the blood glucose measurement values, the insulin dosage data, the health data entries, or combinations thereof via the communication circuit.
3. The diabetes health management system of embodiment 1 wherein the therapy module causes the processor to generate an emergency alarm based at least in part on the blood glucose measurement values.
4. The diabetes health management system of embodiment 3 wherein the communications module further causes the processor to wirelessly transmit the emergency alarm to a caregiver via the communication circuit.
5. The diabetes health management system of embodiment 1 wherein the therapy advice message comprises a basal rate recommendation and a bolus delivery recommendation.
6. The diabetes health management system of embodiment 1 wherein:
   the plurality of user devices comprises an insulin pump; and
   the communications module further causes the processor to control the communication circuit to transmit wireless command signals to the insulin pump such that the portable device is operable to remotely control the insulin pump.
7. The diabetes health management system of embodiment 6 wherein the wireless command signals correspond to a basal rate profile, a basal interval, a basal rate lag time, bolus delivery velocity, bolus initiation, or combinations thereof.
8. The diabetes health management system of embodiment 6 wherein the therapy module comprises a plurality of user definable and selectable basal rate profiles, and the communications module causes the processor to control the communication circuit to transmit the wireless command signals to the insulin pump in accordance with a user selected basal rate profile.
9. The diabetes health management system of embodiment 8 wherein the program code provides a warning if the user selects an empty basal rate profile from the plurality of basal rate profiles.
10. The diabetes health management system of embodiment 1 wherein
   the plurality of user devices comprises an insulin pump;
   the health data entries comprises food data entries; and
   the therapy module comprises a user selectable adaptive bolus that when initiated by the program code wirelessly transmits a command signal to the insulin pump that modulates a postprandial insulin infusion based at least in part on a most recent food data entry.
11. The diabetes health management system of embodiment 1 wherein:
   the user interface is operable to receive the health data entries provided by the user;
   the health data entries comprise medication data entries, physical activity data, health state data, and food data; and
   the health state data corresponds to an illness, menstrual state, stress level, or combinations thereof.
12. The diabetes health management system of embodiment 11 wherein:
   the portable device further comprises an accelerometer capable of providing an acceleration signal in accordance with movement of the portable device; and
   the program causes the processor to receive the acceleration signal from the accelerometer and the program code causes the processor to determine the physical activity undertaken by the user from the acceleration signal.
13. The diabetes health management system of embodiment 11 wherein:
   the data module further comprises a food database comprising a plurality of user selectable food items, each food item having at least one nutritional attribute;
   the food data comprises a plurality of food entries, each food entry corresponding with a selected food item; and
   the data module causes the processor to receive and store in the memory the selected food item and a corresponding serving size.
14. The diabetes health management system of embodiment 11 wherein the data module further causes the processor to:
   receive a photograph of a food item consumed by the user, associate the photograph with the food item and store the photograph in the memory; and
   receive a voice recording describing the food item consumed by the user, associate the voice recording with the food item, and store the voice recording in the memory.
15. The diabetes health management system of embodiment 11 wherein:
   the program code causes the processor to determine a location of the user from a location signal provided by the portable device, and to detect a food serving establishment based on the location as determined by the location signal; and
   the therapy module causes the processor to display food selection advice on the user interface based on the food serving establishment, the blood glucose measurement values, the health data entries, or combinations thereof.
16. The diabetes health management system of embodiment 1 wherein:
   the data module causes the processor to store the health data entries provided by the user;
   the health data entries comprise physical activity data corresponding to physical activities undertaken by the user and food data corresponding to food items consumed by the user;
   the food data comprises one or more nutritional attributes associated with each food item consumed by the user;
   the data module instructs the processor to compile a food intake profile based on the food data;
   the data module further instructs the processor to compile an exercise profile based on the physical activities undertaken by the user and blood glucose values measured after each physical activity; and
   the therapy advice message is based on the food intake profile and the exercise profile.
17. The diabetes health management system of embodiment 1 wherein:
   the program code causes the processor to determine a location of the user from a location signal provided by the portable device;
   the health data entries and blood glucose measurement data are associated with the location of the user as provided by the location signal;
   the data module causes the processor to generate a location profile comprising the associated health data entries for one or more locations visited by the user; and
   the therapy module causes the processor to display a therapy advice message based on the location profile associated with a current location of the user on the user interface.
18. The diabetes health management system of embodiment 1 wherein:
   the program code causes the processor to determine a location of the user from a location signal provided by the portable device and detect a time zone change from the location signal; and
   the therapy module causes the processor to alter the therapy advice message such that a therapy is incrementally shifted from a first time zone therapy schedule to a second time zone therapy schedule.
19. The diabetes health management system of embodiment 18 wherein the therapy module incrementally shifts the therapy from the first time zone therapy schedule to the second time zone therapy schedule in accordance with a user defined time zone change profile.
20. The diabetes health management system of embodiment 1 wherein:
   the portable device is configured to operate in a plurality of user selectable life modes; and
   each life mode is operable to adjust the therapy advice message.
21. The diabetes health management system of embodiment 1 wherein:
   the blood glucose measuring device comprises a blood glucose meter; and
   the therapy module causes the processor to display a blood glucose measurement reminder on the user interface based on the health data entries.
22. The diabetes health management system of embodiment 21 wherein the therapy module causes the processor to display blood glucose reminders on the user interface in accordance with a hypoglycemia reminder schedule if the user enters a hypoglycemic state, and display blood glucose reminders on the user interface in accordance with a hyperglycemic reminder schedule if the user enters a hyperglycemic state.
23. The diabetes health management system of embodiment 1 wherein the graphical representation depicts data related to at least one event, the event comprising a hypoglycemic state, a hyperglycemic state, a physical activity, an illness, or a stress level.
24. The diabetes health management system of embodiment 1 wherein the analysis module causes the processor to:
   display on the user interface a hypoglycemic state report comprising the blood glucose measurements, the insulin dosage data and the health data entries proximate a hypoglycemic state; and
   display on the user interface a hyperglycemic state report comprising the blood glucose measurements, the insulin dosage data and the health data entries proximate a hyperglycemic state.
25. A diabetes health management system for use in a portable device having a global positioning module capable of providing a global positioning signal, a processor, a memory and a user interface, the diabetes health management system comprising program code further comprising a data module and a therapy advice module, wherein:
   the program code causes the processor to determine a present location of a user from the global positioning signal;
   the data module causes the processor to receive and store health data in the memory, and generate and store a plurality of location profiles in the memory, each location profile comprising the health data associated therewith; and
   the therapy advice module causes the processor to display a therapy advice message on the user interface of the portable device, wherein the therapy advice message is based on a location profile corresponding with the present location of the user.
26. The diabetes health management system of embodiment 25 wherein the health data comprises blood glucose measurement data, insulin dosage data, physical activity data, and food intake data.
27. The diabetes health management system of embodiment 26 wherein the data module further causes the processor to add health data received while the user is located at the present location to the location profile associated with the present location.
28. The diabetes health management system of embodiment 26 wherein:
   the plurality of location profiles comprises a work location profile and a home location profile;
   the work location profile comprises health data received by the data module while the user is located at a work location, and the therapy advice message provided to the user while the user is at the work location is based on the work location profile; and
   the home location profile comprises health data received by the data module while the user is located at a home location, and the therapy advice message provided to the user while the user is at the home location is based on the home location profile.
29. The diabetes health management system of embodiment 25 wherein:
   the program code causes the processor to detect if the user has moved from a first time zone to a second time zone by comparing the present location with a recent location; and
   the therapy advice message causes the processor to incrementally shift an insulin therapy from a first time zone therapy schedule corresponding to the first time zone to a second time zone therapy schedule corresponding to the second time zone.
30. The diabetes health management system of embodiment 29 wherein the insulin therapy is incrementally shifted from the first time zone therapy schedule to the second time zone therapy schedule in accordance with a user defined time zone change profile.

The following is a list of possible methods, which can be performed by the mentioned embodiments of the invention, i.e. the embodiments comprises means for carrying out the steps of these methods:
1. A method of providing diabetes therapy to a user via a portable device, the method comprising:
   receiving blood glucose measurement values from a blood glucose measurement device;
   receiving insulin dosage data from an insulin administration device;
   receiving health data entries provided by the user;
   storing the blood glucose measurement values, the insulin dosage data, and the health data entries in a memory location of the portable device;
   generating a report comprising information selected from the blood glucose measurement values, the insulin dosage data, the health data entries, or combinations thereof; and
   wirelessly transmitting the report to a caregiver.
2. The method according to method 1 wherein the blood glucose measurement values, insulin dosage data and health data entries are wirelessly transmitted to a remote data storage location.
3. The method according to method 1 wherein the health data entries comprise medication data entries, physical activity data entries, health state data entries, and food data entries.
4. The method according to method 1 wherein:
   the report corresponds to an event determined by the blood glucose measurement values, the health data entries, or a combination thereof; and
   the report comprises blood glucose measurement data, insulin dosage data, and health data entries during a reporting time range comprising a pre-event period and a post-event period.
5. The method according to method 1 wherein the method further comprises prompting the user to perform a blood glucose measurement test.
6. The method according to method 1 wherein the method further comprises:
   receiving a scheduled event entry; and
   prompting the user to perform a plurality of blood glucose measurement tests in accordance with a testing schedule associated with the scheduled event entry.

## Claims

1. A diabetes health management system comprising an insulin administrating device and a portable device, wherein the portable device has a user interface, a processor, and a memory, wherein the diabetes health management system comprising a program code which comprises a data module, a therapy module, and an analysis module, wherein:
the data module causes the processor of the portable device to receive and store blood glucose measurement values which has been associated with detected location information, insulin dosage data from the insulin administrating device, and health data entries into the memory;
the therapy module causes the processor to determine a predicted user behavior based at least in part on the blood glucose measurement values, the detected location information, the insulin dosage data, and the health data entries stored in the memory, and display a therapy advice message based on the determined predicted user behavior on the user interface; in particular wherein the therapy advice message comprises a basal rate recommendation and a bolus delivery recommendation and
the analysis module causes the processor to generate and display to a user on the user interface a graphical representation of selected blood glucose measurement values, selected insulin dosage data, selected health data entries, or combinations thereof.

2. The diabetes health management system of claim 1, wherein the portable device comprises a communication circuit and the program code comprises a communications module, so that the communications module causes the processor to control the communication circuit to wirelessly couple the portable device to a plurality of user devices which in particular comprise an insulin pump, and display information provided by the plurality of user devices on the user interface; in particular wherein the communications module further causes the processor to transmit to a health network server the blood glucose measurement values, the insulin dosage data, the health data entries, or combinations thereof via the communication circuit.

3. The diabetes health management system of claim 1, or 2, wherein the therapy module causes the processor to generate an emergency alarm based at least in part on the blood glucose measurement values, wherein preferably the communications module further causes the processor to wirelessly transmit the emergency alarm to a caregiver via the communication circuit.

4. The diabetes health management system of claim 2 wherein the communications module further causes the processor to control the communication circuit to transmit wireless command signals to the insulin pump such that the portable device is operable to remotely control the insulin pump, wherein preferably the wireless command signals correspond to a basal rate profile, a basal interval, a basal rate lag time, bolus delivery velocity, bolus initiation, or combinations thereof.

5. The diabetes health management system of claim 2 or 4 wherein the therapy module comprises a plurality of user definable and selectable basal rate profiles, and the communications module causes the processor to control the communication circuit to transmit the wireless command signals to the insulin pump in accordance with a user selected basal rate profile wherein preferably the program code provides a warning if the user selects an empty basal rate profile from the plurality of basal rate profiles.

6. The diabetes health management system of any of claim 4 to 5 wherein
the health data entries comprises food data entries; and
the therapy module comprises a user selectable adaptive bolus that when initiated by the program code wirelessly transmits a command signal to the insulin pump that modulates a postprandial insulin infusion based at least in part on a most recent food data entry.

7. The diabetes health management system of any of claim 1 to 6 wherein:
the user interface is operable to receive the health data entries provided by the user;
the health data entries comprise medication data entries, physical activity data, health state data, and food data; and
the health state data corresponds to an illness, menstrual state, stress level, or combinations thereof.

8. The diabetes health management system of any of claim 1 to 7 wherein:
the portable device further comprises an accelerometer capable of providing an acceleration signal in accordance with movement of the portable device; and
the program causes the processor to receive the acceleration signal from the accelerometer and the program code causes the processor to determine the physical activity undertaken by the user from the acceleration signal.

9. The diabetes health management system according to any preceding claim wherein:
the data module further comprises a food database comprising a plurality of user selectable food items, each food item having at least one nutritional attribute;
the food data comprises a plurality of food entries, each food entry corresponding with a selected food item; and
the data module causes the processor to receive and store in the memory the selected food item and a corresponding serving size.

10. The diabetes health management system according to any preceding claim wherein the data module further causes the processor to:
receive a photograph of a food item consumed by the user, associate the photograph with the food item and store the photograph in the memory; and/or
receive a voice recording describing the food item consumed by the user, associate the voice recording with the food item, and store the voice recording in the memory.

11. The diabetes health management system according to any preceding claim wherein:
the program code causes the processor to determine the location information from a location signal provided by the portable device, and to detect a food serving establishment based on the location as determined by the location signal; and
the therapy module causes the processor to display food selection advice on the user interface based on the food serving establishment, the blood glucose measurement values, the health data entries, or combinations thereof.

12. The diabetes health management system according to any preceding claim wherein:
the data module causes the processor to store the health data entries provided by the user;
the health data entries comprise physical activity data corresponding to physical activities undertaken by the user and food data corresponding to food items consumed by the user;
the food data comprises one or more nutritional attributes associated with each food item consumed by the user;
the data module instructs the processor to compile a food intake profile based on the food data;
the data module further instructs the processor to compile an exercise profile based on the physical activities undertaken by the user and blood glucose values measured after each physical activity; and
the therapy advice message is based on the food intake profile and the exercise profile.

13. The diabetes health management system according to any preceding claim wherein:
the program code causes the processor to determine the location information from a location signal provided by the portable device;
the health data entries and blood glucose measurement data are associated with the location information as provided by the location signal;
the data module causes the processor to generate a location profile comprising the associated health data entries for one or more locations visited by the user; and
the therapy module causes the processor to display a therapy advice message based on the location profile associated with a current location of the user on the user interface.

14. The diabetes health management system of any of claim 1 to 12 wherein:
the program code causes the processor to determine the location information from a location signal provided by the portable device and detect a time zone change from the location signal; and
the therapy module causes the processor to alter the therapy advice message such that a therapy is incrementally shifted from a first time zone therapy schedule to a second time zone therapy schedule wherein preferably the therapy module incrementally shifts the therapy from the first time zone therapy schedule to the second time zone therapy schedule in accordance with a user defined time zone change profile.

15. The diabetes health management system according to any of claim 13 or 14 wherein the portable device comprises a global positioning module capable of providing as the location signal a global positioning signal, wherein preferably the data module further causes the processor to add blood glucose measurement data, insulin dosage data, and health data received while the user is located at the present location to the location profile associated with the present location, in particular wherein:
the plurality of location profiles comprises a work location profile and a home location profile;
the work location profile comprises health data received by the data module while the user is located at a work location, and the therapy advice message provided to the user while the user is at the work location is based on the work location profile; and
the home location profile comprises health data received by the data module while the user is located at a home location, and the therapy advice message provided to the user while the user is at the home location is based on the home location profile.
